# EUROPEAN PATENT SPECIFICATION

(11) **EP 0 410 433 B1**
(45) Date of publication and mention of the grant of the patent: **28.02.1996**
(21) Application number: 90114291.9
(22) Date of filing: 25.07.1990
(51) Int. Cl.: C07H 17/08, C07H 17/00, A61K 31/70

(54) **Tylosin derivatives**
Tylosin-Derivate
Dérivés de tylosine

(30) Priority: 26.07.1989 YU 1498/89
(43) Date of publication of application: 30.01.1991
(73) Proprietor: PLIVA FARMACEUTSKA, KEMIJSKA, PREHRAMBENA I KOZMETICKA INDUSTRIJA S P.O., YU-41001 Zagreb (YU)
(72) Inventor: Lopotar, Nevenka, Yu-41000 Zagreb (YU); Djokic, Slobodan, Dr., YU-41000 Zagreb (YU)
(74) Representative: von Füner, Alexander, Dr.

(56) References cited:
- EP-A- 0 109 253
- EP-A- 0 287 082
- US-A- 3 178 341

## Description

The present invention relates to new tylosin derivatives, to a method for the preparation thereof and their use in the preparation of pharmaceuticals, especially as valuable intermediates in the synthesis of 17-membered azalide antibiotics.

Tylosin is a 16-membered macrolide antibiotic prepared by means of fermentation and is described in Tetrahedron Letters 2339, 1970 and in U.S. Patent 3,178,341.

It is known that a series of imino tylosin derivatives have been prepared by chemical transformation of C-9 keto and/or C-20 aldehyde groups including tylosin oxime, 4'-demicarosyltylosin oxime, relomycin oxime and 10,11,12,13-tetrahydro derivatives and the corresponding dimethylacetal derivatives thereof (unexamined YU 674/87 or EP 0 287 082 A2).

It is known that Beckmann rearrangement of oximes in the presence of protonic or Lewis acids or *via* the *in situ* prepared O-arylsulfonates of ketoxime in the presence of water yields amides or - starting from cyclic systems - lactams (Org. React. 11, 1, 1960: Houben-Weyl Bd. VII/2b, 1986, 1976; J.Chem.Soc. 1948, 1518). The arylsulfochloride-base method is the most appropriate for the performance of Beckmann rearrangement of unstable oximes (J.Am.Chem.Soc. 87, 5646, 1965).

In the hitherto known prior art in the field of 16-membered macrolides of the tylosin series, the Beckmann rearrangement of tylosin oxime and derivatives thereof has not been described as yet.

The new tylosin derivatives (I) of the invention are represented by the formula 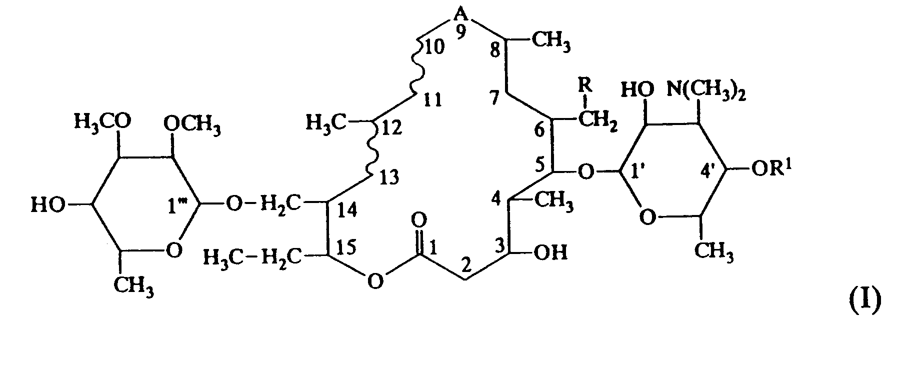
mycarosyl 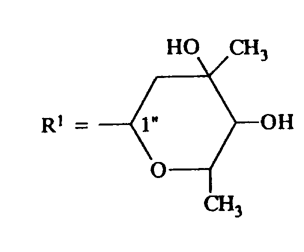
wherein A stands for a -CO-NH- or a -NH-CO- group, R stands for -CHO, -CH(OCH₃)₂ or CH₂OH, R¹ stands for mycarosyl or hydrogen and 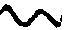 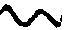 has the meaning of a double or a single bond.

It has been found that tylosin derivatives of the formula I 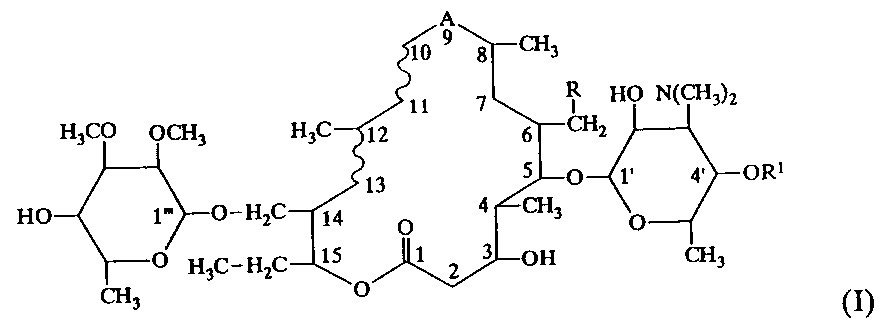
mycarosyl 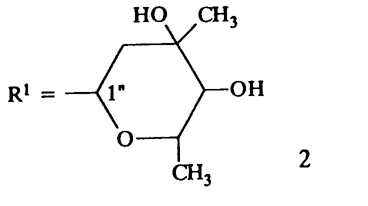
wherein

| | | | | |
|---|---|---|---|---|
| Ia | A = CO-NH, | R = CH(OCH₃)₂, | R¹ = mycarosyl, | 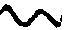 = double bond, |
| Ib | A = CO-NH, | R = CHO, | R¹ = mycarosyl, | 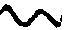 = double bond, |
| Ic | A = CO-NH, | R = CH(OCH₃)₂, | R¹ = H, | 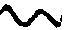 = double bond, |
| Id | A = CO-NH, | R = CHO, | R¹ = H, | 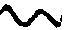 = double bond, |
| Ie | A = CO-NH, | R = CH₂OH, | R¹ = mycarosyl, | 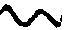 = double bond, |
| Ie' | A = NH-CO, | R = CH₂OH, | R¹ = mycarosyl, | 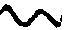 = double bond, |
| If | A = CO-NH, | R = CH₂OH, | R¹ = mycarosyl, | 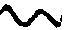 = single bond, |
| If' | A = NH-CO, | R = CH₂OH, | R¹ = mycarosyl, | 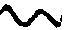 = single bond, |
| Ig | A = CO-NH, | R = CH(OCH₃)₂, | R¹ = mycarosyl, | 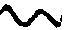 = single bond, |
| Ih | A = CO-NH, | R = CHO, | R¹ = mycarosyl, | 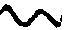 = single bond, |
| Ii | A = CO-NH, | R = CHO, | R¹ = H, | 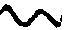 = single bond, |
| Ij | A = CO-NH, | R = CH₂OH, | R¹ = H, | 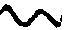 = double bond, |
| Ij' | A = NH-CO, | R = CH₂OH, | R¹ = H, | 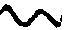 = double bond, |
| Ik | A = CO-NH, | R = CH₂OH, | R¹ = H, | 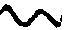 = single bond, |
| Ik' | A = NH-CO, | R = CH₂OH, | R¹ = H, | 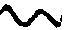 = single bond, |

may be obtained by means of following processes:

### process A/

comprising the Beckmann rearrangement of compounds of the formula II 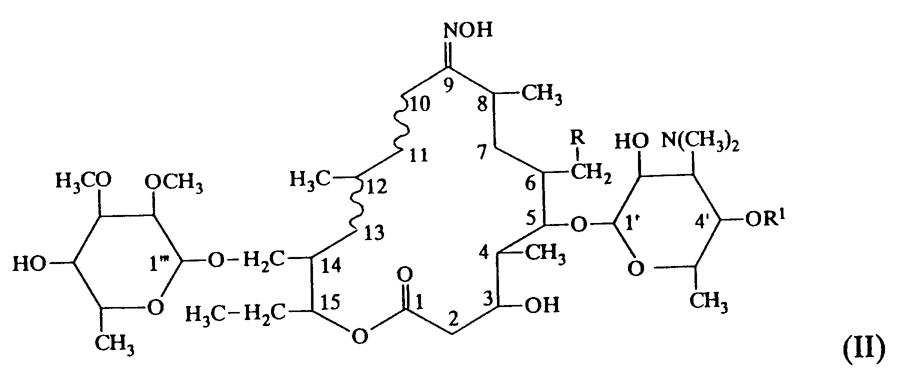 mycarosyl 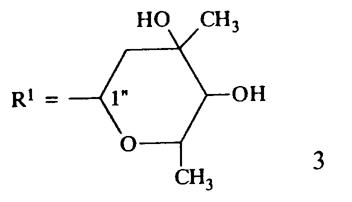 wherein

| | | | |
|---|---|---|---|
| IIa | R = CH(OCH₃)₂, | R¹ = mycarosyl, | 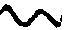 = double bond, |
| IIb | R = CHO, | R¹ = mycarosyl, | 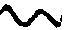 = double bond, |
| IIc | R = CH(OCH₃)₂, | R¹ = H, | 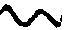 = double bond, |
| IId | R = CHO, | R¹ = H, | 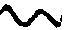 = double bond, |
| IIe | R = CH₂OH, | R¹ = mycarosyl, | 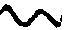 = double bond, |
| IIf | R = CH₂OH, | R¹ = mycarosyl, | 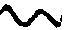 = single bond, |
| IIg | R = CH(OCH₃)₂, | R¹ = mycarosyl, | 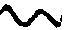 = single bond, |
| IIh | R = CHO, | R¹ = mycarosyl, | 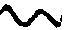 = single bond, |

with aromatic sulfochlorides of the formula 4-R-C₆H₄-SO₂Cl, wherein R represents alkyl, halo or an acylamino group, in the presence of organic or inorganic bases, yielding compounds of the formula I 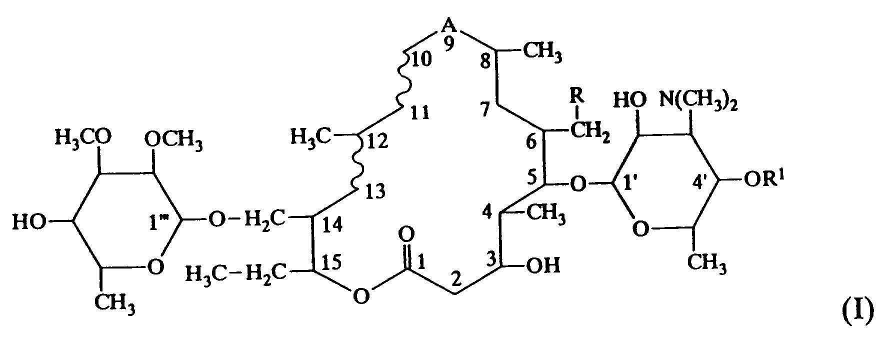 mycarosyl 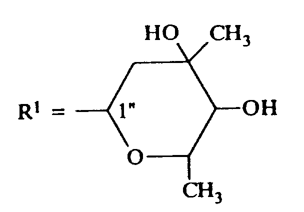 wherein

| | | | | |
|---|---|---|---|---|
| Ia | A = CO-NH, | R = CH(OCH₃)₂, | R¹ = mycarosyl, | 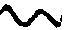 = double bond, |
| Ib | A = CO-NH, | R = CHO, | R¹ = mycarosyl, | 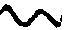 = double bond, |
| Ic | A = CO-NH, | R = CH(OCH₃)₂, | R¹ = H, | 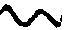 = double bond, |
| Id | A = CO-NH, | R = CHO, | R¹ = H, | 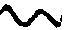 = double bond |
| Ie | A = CO-NH, | R = CH₂OH, | R¹ = mycarosyl, | 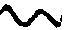 = double bond |
| Ie' | A = NH-CO, | R = CH₂OH, | R¹ = mycarosyl, | 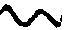 = double bond |
| If | A = CO-NH, | R = CH₂OH, | R¹ = mycarosyl, | 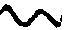 = single bond |
| If' | A = NH-CO, | R = CH₂OH, | R¹ = mycarosyl, | 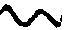 = single bond |
| Ig | A = CO-NH, | R = CH(OCH₃)₂, | R¹ = mycarosyl, | 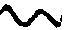 = single bond, |
| Ih | A = CO-NH, | R = CHO, | R¹ = mycarosyl, | 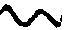 = single bond; |

### process B/

comprising the hydrolysis of compounds of formula I 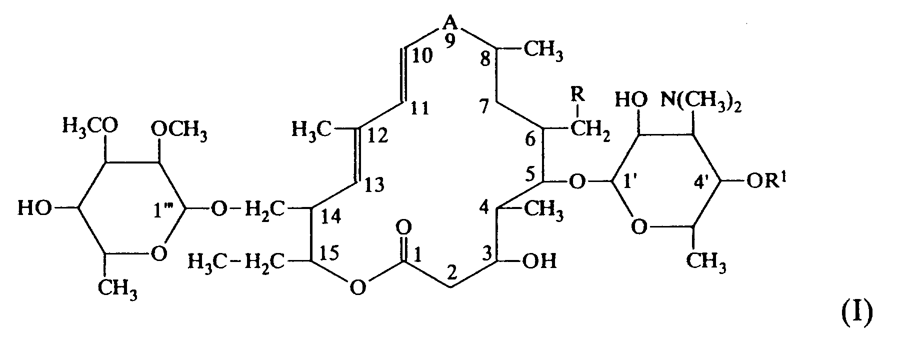 mycarosyl 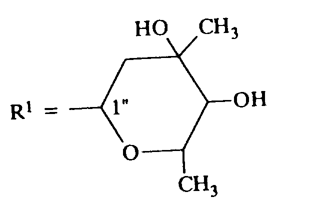 wherein

| | | | |
|---|---|---|---|
| Ia | A = CO-NH, | R = CH(OCH₃)₂, | R¹ = mycarosyl, |
| Ic | A = CO-NH, | R = CH(OCH₃)₂, | R¹ = H, |
| Ie | A = CO-NH, | R = CH₂OH, | R¹ = mycarosyl, |
| Ie' | A = NH-CO, | R = CH₂OH, | R¹ = mycarosyl, |

by means of a catalytical amount of organic acids in aprotic solvents in the presence of water or with inorganic acids in the presence of aprotic solvents, yielding compounds of the formula I 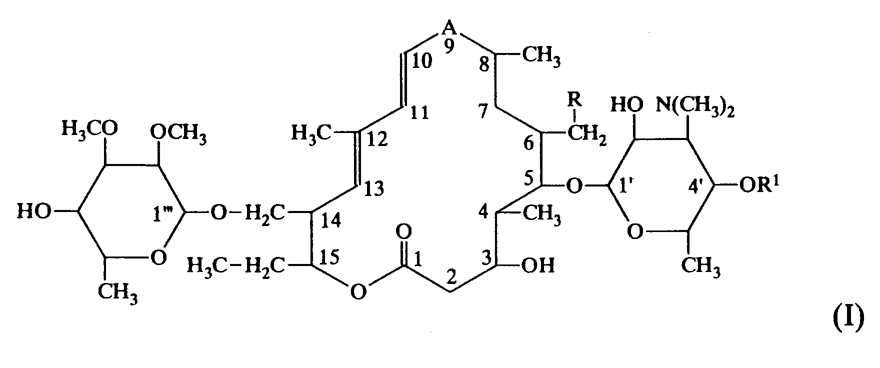 mycarosyl 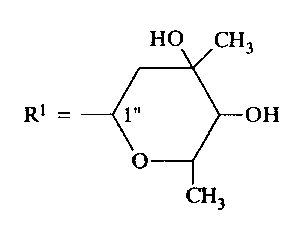
**wherein**

| | | | |
|---|---|---|---|
| Ib | A = CO-NH, | R = CHO, | R¹ = mycarosyl, |
| Id | A = CO-NH, | R = CHO, | R¹ = H, |
| Ij | A = CO-NH, | R = CH₂OH, | R¹ = H, |
| Ij' | A = NH-CO, | R = CH₂OH, | R¹ = H; |

### process C/

comprising the catalytical hydrogenation of compounds of formula I 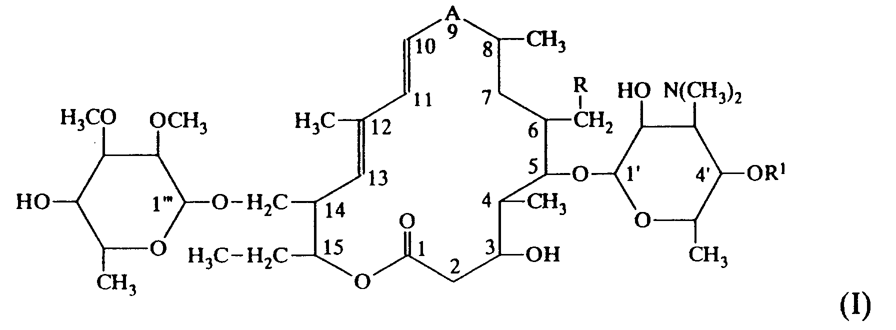 mycarosyl 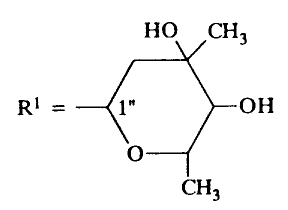 wherein

| | | | |
|---|---|---|---|
| Ia | A = CO-NH, | R = CH(OCH₃)₂, | R¹ = mycarosyl, |
| Ib | A = CO-NH, | R = CHO, | R¹ = mycarosyl, |
| Ie | A = CO-NH, | R = CH₂OH, | R¹ = mycarosyl, |
| Ie' | A = NH-CO, | R = CH₂OH, | R¹ = mycarosyl, |
| Ij | A = CO-NH, | R = CH₂OH, | R¹ = H, |
| Ij' | A = NH-CO, | R = CH₂OH, | R¹ = H, |

with H₂ in the presence of noble metal catalysts in inert solvents, yielding compounds of the formula I 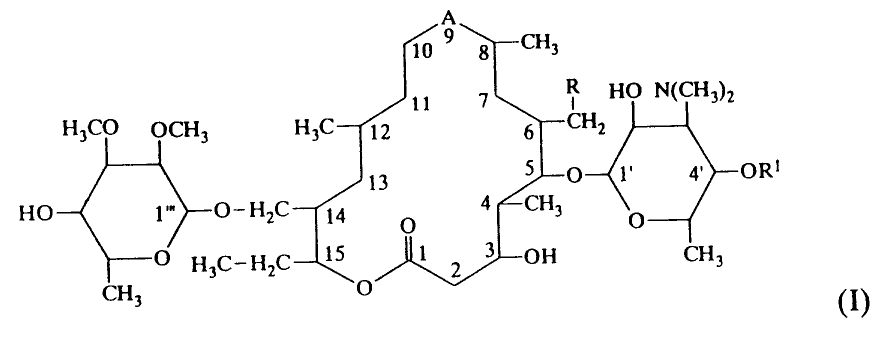 mycarosyl 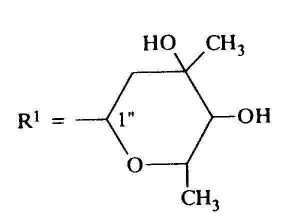 wherein

| | | | |
|---|---|---|---|
| If | A = CO-NH, | R = CH₂OH, | R¹ = mycarosyl, |
| If' | A = NH-CO, | R = CH₂OH, | R¹ = mycarosyl, |
| Ig | A = CO-NH, | R = CH(OCH₃)₂, | R¹ = mycarosyl, |
| Ih | A = CO-NH, | R = CHO, | R¹ = mycarosyl, |
| Ii | A = CO-NH, | R = CHO, | R¹ = H, |
| Ik | A = CO-NH, | R = CH₂OH, | R¹ = H, |
| Ik' | A = NH-CO, | R = CH₂OH, | R¹ = H. |

According to process A/ of the present invention the reaction of Beckmann rearrangement of oximes (IIa-IIh) is performed with 1-4 moles of aromatic sulfochlorides of the formula III

4-R-C₆H₄-SO₂Cl (III)

wherein R stands for C₁-C₃ alkyl, halo or an acylamino group of the formula IV

-NH-COR₁ (IV)

wherein R₁ represents methyl, and with 1-5 moles of inorganic bases (NaHCO₃, NaOH) or organic bases (pyridine, triethylamine) at a temperature from 0-5 °C to room temperature, in a period of 30 minutes to 6 hours, in a mixture of a lower ketone (acetone, methylethyl ketone) and water. After the completed reaction the solvent is evaporated at reduced pressure and the products (Ia-Ih) are isolated by means of gradient pH extraction with halogenated hydrocarbons e.g. chloroform or methylene chloride.

According to process B/ of the present invention the hydrolysis of the compounds (Ia, Ic) is performed in 50% acetonitrile in the presence of a catalytical amount of an organic acid such as trifluoroacetic acid, at room temperature in a period from 30 minutes to 24 hours, whereas the hydrolysis of compounds (Ie and Ie') is performed in the presence of a catalytical amount of an inorganic acid such as e.g. hydrochloric or sulfuric acid. The isolation of the products (Ib, Id, Ij, Ij') is performed by means of extraction from aqueous-alkaline solutions with halogenated solvents and evaporation to dryness.

According to process C/ of the present invention the catalytical hydrogenation of the compounds (Ia, Ib, Ie, Ie', Ij, Ij') is performed at a hydrogen pressure of 0.2 to 2.0 MPa in the presence of a noble metal catalyst, in inert solvents at room temperature in a period of 2 to 6 hours. Suitable catalysts are palladium-on-carbon, platinum-on-carbon, rhodium-on-carbon,PtO₂ or Raney nickel, whereas suitable solvents are lower (C₁-C₄) alcohols, ethylacetate, diethylether or tetrahydrofurane. Upon completed reaction the catalyst is filtered off and the products (If, If',Ig, Ih, Ii, Ik, Ik') are isolated by means of evaporation to dryness.

The structure of the compounds is confirmed by spectrometric (IR, UV, ¹H NMR, ¹³C NMR) analysis and mass analysis.

The new tylosin derivatives exhibit a biological action and a practical utility as intermediates in the syntesis of 17-membered azalide antibiotics, as yet unknown in the chemical and biological practice.

The compounds of the present invention may be defined as 1-oxa-9-aza-cycloheptadecane-8,17-dione-3[[(6-deoxy-2,3-di-O-methyl-βD-alopyranosyl)oxy]methyl]-13-[[3,6-dideoxy-4-O-(2,6-dideoxy-3-C-methyl-α-L-ribo-hexo-pyranosyl)-3-(dimethylamino)-β-D-glucopyranosyl]oxy]-2-ethyl-15-hydroxy-5,10,14-trimethyl-4,6-diene-12-acetaldehydes or alternatively as aza-homotylosins (IUPAC Nomenclature of Organic Chemistry, 1979 Edition, Pergamon Press, New York, 491-511).

According to the above nomenclature the present compounds are named:
Ib 8a-aza-8a-homotylosin,
Id 4'-demicarosyl-8a-aza-8a-homotylosin,
Ih 8a-aza-8a-homo-10,11,12,13-tetrahydrotylosin,
Ii 4'-demicarosyl-8a-aza-8a-homo-10,11,12,13-tetrahydrotylosin,
Ia, Ic and Ig (in which R stands for dimethylacetal) are the corresponding dimethylacetals,
Ie 8a-aza-8a-homorelomycin,
Ie' 9a-aza-9a-homorelomycin,
Ij and Ij' are the 4'-demicarosyl derivatives, whereas the compounds If, If', Ik and Ik' are the corresponding 10,11,12,13-tetrahydro derivatives.

The invention is illustrated by the following Examples:

### Example 1

### 8a-Aza-8a-homotylosin dimethylacetal (Ia)

Solutions of p-toluenesulfonyl chloride (0.40 g; 2.10 mmole) in acetone (10 mL) and NaHCO₃ (0.30 g; 3.57 mmole) in water (37 mL) are added dropwise under stirring within 30 minutes to a solution of tylosinoxime dimethylacetal (IIa) (1.0 g; 1.02 mmole) in acetone (15 mL) at a temperature of 0 to 5 °C. After stirring the reaction mixture for 3 hours at said temperature, the acetone is evaporated at reduced pressure; then 10 mL of chloroform are added to the residual aqueous suspension and it is acidified to a pH of 5.5 with 1N HCl. The layers are separated and the aqueous layer is repeatedly extracted with chloroform until pH 5.5 (1x10 mL) and pH 7.5 (3x20 mL) are reached. The chloroform extracts are dried on K₂CO₃ and then evaporated to dryness. At pH 7.5 there are isolated 0.72 g (72.0%) of the title product of the following physical-chemical characteristics:
IR (CHCl₃) 3405, 1705, 1650, 1610, 1500 cm⁻¹
- ¹H NMR (CDCl₃) δ ppm: 7.19 (H, d, H-11), 5.85 (H, d, H-10), 5.70 (H, d, H-13), 5.33 (H, d, NH) disappears after exchange with D₂O, 3.61 (3H, s, 3'''OCH₃), 3.47 (3H, s, 2'''OCH₃), 3.34 (6H, s, 2x20-(OCH₃))
- ¹³C NMR (CDCl₃) δ ppm: 173.27 (C-1), 166.42 (C-9, amide C=O), 144.87 (C-11), 138.33 (C-13), 135.00 (C-12), 119.53 (C-10), 104.07 (C-20), 103.56 (C-1'), 101.08 (C-1'''), 96.28 (C-1''), 61.69 (C-3''' OCH₃), 59.60 (C-2'''OCH₃), 53.73 (C-20 OCH₃), 50.92 (C-20 OCH₃)

### Example 2

### 8a-Aza-8a-homotylosin (Ib)

### Method A

Starting from 0.250 g (0.269 mmole) of tylosin-9-oxime (IIb), dissolved in 5 mL of acetone, 0.118 g (0.62 mmole) of p-toluenesulfonyl chloride dissolved in 3 mL of acetone and 0.087 g (1.04 mmole) of NaHCO₃ dissolved in 12 mL of water and working as described in Example 1, there are isolated 0.15 g (60.0 %) of the title product of the following physical-chemical characteristics:
IR (CHCl₃) 3405, 1715, 1665, 1615, 1500 cm⁻¹
- ¹H NMR (CDCl₃) δ ppm: 9.76 (H, s, H-20), 7.19 (H, d, H-11), 5.80 (H, d, H-10), 5.75 (H, d, H-13), 5.39 (H, d, NH) disappears after exchange with D₂O
- ¹³C NMR (CDCl₃) δ ppm: 203.39 (C-20), 173, 43 (C-1), 166.60 (C-9, amide C=O), 145.26 (C-11), 138.26 (C-13), 135.16 (C-12), 119.25 (C-10), 103.33 (C-1'), 101.13 (C-1'''), 96.29 (C-1'')

### Method B

8a-Aza-8a-homotylosin dimethylacetal (Ia) (0.60 g; 0.61 mmole) is dissolved in 20 mL of acetonitrile, whereupon there are added 20 mL of water and 0.11 mL of trifluoroacetic acid. After stirring for 4 hours at room temperature, the solution is made alkaline (pH 8-8.5) by means of addition of a saturated solution of sodium hydrogen carbonate and extracted with chloroform. The combined extracts are washed with water, dried on K₂CO₃ and then evaporated to dryness. 0.45 g of the crude product are purified on a silica gel column /CH₂Cl₂:CH₃OH:conc.NH₄OH (90:9:1.5)/, yielding 0.32 g (56.4 %) of the title product.

### Example 3

### 4'-Demicarosyl-8a-aza-8a-homotylosin dimethylacetal (Ic)

Starting from 1.0 g (1.2 mmole) of 4'-demicarosyl-tylosin-oxime dimethylacetal (IIc) dissolved in 15 mL of acetone, 0.473 g (2.48 mmole) of p-toluenesulfonyl chloride dissolved in 10 mL of acetone and 0.350 g (4.16 mmole) of NaHCO₃ dissolved in 37 mL of water and working as described in Example 1, there are isolated 0.70 g (70.1 %) of the title product of the following physical-chemical characteristics:
IR (CHCl₃) 3400, 1700, 1690, 1645, 1500 cm⁻¹
- ¹H NMR (CDCl₃) δ ppm: 7.14 (H d, H-11), 5.85 (H, d, H-10), 5.64 (H, d, H-13), 5.28 (H, d, NH) disappears after exchange with D₂O, 3.61 (3H, s, 3'''OCH₃), 3.47 (3H, s, 2'''OCH₃), 3.35 (6H, s, 2x20-(OCH₃)), 2.49 (6H, s, N(CH₃)₂), 1.75 (3H, s, H-22)
- ¹³C NMR (CDCl₃) δ ppm: 173.08 (C-1), 166.42 (C-9, amide C=O), 144.81 (C-11), 138.27 (C-13), 134.94 (C-12), 119.47 (C-10), 104.01 (C-20), 103.50 (C-1'), 101.02 (C-1'''), 61.63 (C-3'''OCH₃), 59.54 (C-2'''OCH₃), 53.73 (C-20 OCH₃), 50.92 (C-20 OCH₃)

### Example 4

### 4'-Demicarosyl-8a-aza-8a-homotylosin (Id)

### Method A

Starting from 0.50 g (0.64 mmole) of 4'-demicarosyl-tylosin-oxime (IId) dissolved in 8 mL of acetone, 0.236 g (1.24 mmole) of p-toluenesulfonyl chloride dissolved in 5 mL of acetone and 0.175 g (2.08 mmole) of NaHCO₃ dissolved in 22 mL of water and working as described in Example 1, there are isolated 0.38 g (76.0 %) of the product of the following physical-chemical characteristics:
IR (CHCl₃) 3400, 1705, 1690, 1640, 1595 cm⁻¹
- ¹H NMR (CDCl₃) δ ppm: 9.76 (H, s, H-20), 7.19 (H, d, H-11), 5.91 (H, d, H-10), 5.75 (H, d, H-13), 5.47 (H, d, NH) disappears after exchange with D₂O, 3.61 (3H, s, 3'''OCH₃), 3.49 (3H, s, 2'''OCH₃), 2.50 (6H, s, N(CH₃)₂), 1.75 (3H, s, H-22)
- ¹³C NMR (CDCl₃) δ ppm: 204.12 (C-20), 173.48 (C-1), 166.93 (C-9, amide C=O), 145.09 (C-11), 138.32 (C-13), 135.11 (C-12), 119.53 (C-10), 103.73 (C-1'), 101.07 (C-1''')

### Method B

4'-Demicarosyl-8a-aza-8a-homotylosin dimethylacetal (Ic) (0.40 g; 0.48 mmole) is dissolved in 15 mL of acetonitrile, whereupon there are added 15 mL of water and 0.075 mL of trifluoroacetic acid; after working as described in Example 2, Method B, there are isolated 0.35 g (92.6 %) of the title product.

### Example 5

### 8a-Aza-8a-homorelomycin (Ie) and 9a-Aza-9a-homorelomycin (Ie')

Starting from 3.73 g (4.0 mmole) of relomyein-oxime (IIe) dissolved in 30 mL of acetone, 1.525 g (8.0 mmole) of p-toluenesulfonyl chloride dissolved in 30 mL of acetone and 1.14 g (13.57 mmole) of NaHCO₃ dissolved in 90 mL of water and working as described in Example 1, there are at pH 5.5 obtained 2.6 g of the crude product (Ie'), which subsequently to purification on a silica gel column /CH₂Cl₂:CH₃OH:conc.NH₄OH (90:9:1.5)/shows the following physical-chemical characteristics:
IR (CHCl₃) 1690, 1660, 1625 cm⁻¹
UV (EtOH)λₘₐₓ 270 nm logε 4.4
- ¹H NMR (CDCl₃) δ ppm: 8.55 (H, d, NH) disappears after exchange with D₂O
- ¹³C NMR (CDCl₃) δ ppm: 175.67 (C-1), 174.77 (C-9, amide C=O), 135.16 (C-12), 127.99 (C-11), 122.18 (C-13), 117.55 (C-10), 104.01 (C-1'), 101.18 (C-1'''), 96.45 (C-1''), 60.84 (C-20)
M⁺ 932
0.9 g of a crude product (Ie) are isolated at pH 7.5; subsequently to purification on a silica gel column in said system the product shows the following physical-chemical characteristics:
IR (CHCl₃) 3400, 1700, 1640, 1600 cm⁻¹
UV (EtOH)λₘₐₓ 262 nm logε 4.34
- ¹H NMR (CDCl₃) δ ppm: 5.66 (H, b, NH) disappears after exchange with D₂O
- ¹³C NMR (CDCl₃) δ ppm: 173.71 (C-1), 167.50 (C-9, amide C=O), 145.72 (C-11), 138.60 (C-13), 135.10 (C-12), 119.08 (C-10), 103.50 (C-1'), 101.03 (C-1'''), 96.28 (C-1''), 60.05 (C-20)
M⁺ 932

### Example 6

### 8a-Aza-8a-homo- 10,11,12,13-tetrahydrorelomycin (If)

### Method A

Solutions of p-toluenesulfonyl chloride (0.47 g; 2.49 mmole) in acetone (10 mL) and NaHCO₃ (0.35 g; 4.16 mmole) in water (37 mL) are added within 30 minutes dropwise under stirring to a solution of 10,11,12,13-tetrahydrorelomycin oxime (11f) (1.29 g; 1.38 mmole) in acetione (15 mL) at a temperature from 0 to 5 °C. After stirring the reaction mixture for further 5 hours at said temperature, the acetone is evaporated; 20 mL of dichloromethane are then added to the residual suspension and the pH is adjusted to 7.6 with 1N HaOH. Two further extractions with dichloromethane (20 mL) are performed, the combined extracts are then dried on K₂CO₃ and evaporated to dryness, yielding 1.04 g of the crude product. Upon purification on a silica gel column /CH₂Cl₂:CH₃OH:conc.NH₄OH (90:9:1.5)/, there are obtained 0.94 g (72.9 %) of the title product of the following physical-chemical characteristics:
IR (CHCl₃) 1700, 1640 cm⁻¹
- ¹H NMR (CDCl₃) δ ppm: 5.52 (H, d, NH) disappears after exchange with D₂O, 3.60 (3H, s, 3'''OCH₃), 3.52 (3H, s, 2'''OCH₃), 2.48 (6H, s, N(CH₃)₂)
- ¹³C NMR (CDCl₃) δ ppm: 173.31 (C-1), 171.50 (C-9, amide C=O), 104.40 (C-1'), 100.79 (C-1'''), 96.50 (C-1''), 60.32 (C-20)
UV (EtOH)λₘₐₓ 262 nm logε 2.71
M⁺ 936

### Method C

8a-Aza-8a-homorelomycin, Ie, (0.70 g; 0.75 mmole) is dissolved in 20 mL ethanol (96%), 0.05 g of 10% palladium-on-carbon are added and it is hydrogenated for 2 hours at room temperature and a hydrogen pressure of 2.0 MPa. After the filtration of the catalyst and the evaporation of the filtrate to dryness, there are obtained 0.65 g (92.6%) of the product, which after purification on a silica gel column is identical with the product obtained according to Method A.

### 9a-Aza-9a-homo-10,11,12,13-tetrahydrorelomycin (If')

9a-Aza-9a-homorelomycin, Ie', (0.25 g; 0.27 mmole) is dissolved in 15 mL of methanol and upon addition of 0.05 g of 10% palladium-on-carbon it is hydrogenated for 6 hours at room temperature and a hydrogen pressure of 0.3 MPa. The crude product (0.240 g: 95.7%) is purified on a silica gel column yielding 0.18 g (75.0%) of a product of the following characteristics:
IR (CHCl₃) 3405, 1705, 1655 cm⁻¹
- ¹H NMR (CDCl₃) δ ppm: 6.30 (H, b, NH) disappears after exchange with D₂O, 3.62 (3H, s, 3'''OCH₃), 3.51 (3H, s, 2'''OCH₃), 2.50 (6H, s, N(CH₃)₂)
- ¹³C NMR (CDCl₃) δ ppm: 177.15 (C-9, amide C=O), 173.48 (C-1), 106.97 (C-1'), 101.19 (C-1'''), 96.90 (C-1''), 60.67 (C-20)

### Example 7

### 8a-Aza-8a-homo-10,11,12,13-tetrahydrotylosin dimethylacetal (Ig)

### Method A

Starting from 1.0 g (1.02 mmole) of 10,11,12,13-tetrahydrotylosin oxime dimethylacetal (IIg), 0.40 g (2.10 mmole) of p-toluenesulfonyl chloride and 0.30 g (3.57 mmole) of NaHCO₃ and working as described in Example 1, there are isolated 0.70 g (70.0%) of the title product of the following characteristics:
IR (CHCl₃) 1700, 1650 cm⁻¹
- ¹H NMR (CDCl₃) δ ppm: 5.58 (H, NH) disappears after exchange with D₂O, 3.60 (3H, s, 3'''OCH₃), 3.52 (3H, s, 2'''OCH₃), 3.28 (3H, s, 20-OCH₃), 3.23 (3H, s, 20-OCH₃), 2.50 (6H, s, N(CH₃)₂)
- ¹³C NMR (CDCl₃) δ ppm: 173.95 (C-1), 171.95 (C-9, amide C=O), 103.5 (C-1'), 102.18 (C-20), 100.89 (C-1'''), 96,22 (c-1''), 61.66 (C-3'''OCH₃), 59.61 (C-2'''OCH₃), 53.19 (C-20 OCH₃), 49.75 (C-20 OCH₃)

### Method C

Starting from 0.25 g (0.26 mmole) of 8a-aza-8a-homotylosin dimethylacetal, Ia, and working as described in Example 6, Metod C, by means of hydrogenation with palladium-on-carbon there are obtained 0.16 g (62.7%) of the title product.

### Example 8

### 8a-Aza-8a-homo-10,11,12,13-tetrahydrotylosin (Ih)

### Method A

Starting from 1.0 g (1.07 mmole) of 10,11,12,13-tetrahydrotylosin oxime (IIh), 0.40 g (2.10 mmole) of p-toluenesulfonyl chloride and 0.30 g (3.57 mmole) of NaHCO₃ and working as described in Example 1, there are isolated 0.75 g (75.0%) of the product of the following physical-chemical characteristics:
- ¹H NMR (CDCl₃) δ ppm: 9.69 (H, s, H-20), 5.52 (H,, d, NH) disappears after exchange with D₂O, 3.61 (3H, s, 3'''OCH₃), 3.50 (3H, s, 2'''OCH₃), 2.49 (6H, s, N(CH₃)₂)
- ¹³C NMR (CDCl₃) δ ppm: 203.40 (C-20), 173.30 (C-1), 171.50 (C-9, amide C=O), 103.58 (C-1'), 100.89 (C-3'''), 96.22 (C-1'')

### Method C

0.25 g (0.27 mmole) of 8a-aza-8a-homotylosin, Ib, are worked up as described in Example 6 by means of hydrogenation with palladium-on-carbon. After the purification of 0.22 g of the crude product on a silica gel column, there are obtained 0.16 g (64.0%) of the title product.

### Example 9

### 4'-Demicarosyl-8a-aza-8a-homo-10,11,12,13-tetrahydrotylosin (Ii)

### Method C

0.20 g (0.25 mmole) of 4'-demicarosyl-8a-aza-8a-homotylosin, Id, are dissolved in 15 mL of methanol and upon addition of 0.075 g of palladium-on-carbon it is worked up as described in Example 6, Metod C, by means of hydrogenation. There are obtained 0.180 g (90.0%) of a product of the following characteristics:
IR (CHCl₃) 1715, 1650 cm⁻¹
- ¹H NMR (CDCl₃) δ ppm: 9.72 (H, s, H-20), 5.66 (H, b, NH) disappears after exchange with D₂O, 3.61 (3H, s, 3'''OCH₃), 3.50 (3H, s, 2'''OCH₃), 2.49 (6H, s, N(CH₃)₂)

### Example 10

### 4'-Demicarosyl-8a-aza-8a-homorelomycin (Ij) and 4'-Demicarosyl-9a-aza-9a-homorelomycin (Ij')

### Method B

8a-Aza-8a-homorelomycin, Ie, (0.40 g; 0.43 mmole) is dissolved in 10 mL of a mixture of 0.1 N HCl and CH₃CN (2.5:1) and left standing at room temperature for 24 hours, the isolation of the product is carried out according to the method described in Example 2, Method B. The crude product (0.23 g) is purified on a silica gel column, yielding 0.18 g (53.1%) of the product (lj) of the following characteristics:
IR (CHCl₃) 3400, 1700, 1640, 1600 cm⁻¹
- ¹H NMR (CDCl₃) δ ppm: 5.66 (H, b, NH) disappears after exchange with D₂O
- ¹³C NMR (CDCl₃) δ ppm: 173.71 (C-1), 167.50 (C-9, amide C=O), 145.72 (C-11), 138.60 (C-13), 135.11 (C-12), 119.08 (C-10), 103.50 (C-1'), 101.02 (C-1'''), 60.42 (C-20)
Starting from 0.40 g (0.43 mmole) of 9a-aza-9a-homorelomycin, Ie', there are obtained according to the same metod 0.19 g (56.1%) of the product (Ij') of the following characteristics:
IR (CHCl₃) 1690, 1660, 1625 cm⁻¹
- ¹H NMR (CDCl₃) δ ppm: 8.55 (H, d, NH) disappears after exchange with D₂O
- ¹³C NMR (CDCl₃) δ ppm: 175.79 (C-1), 174.66 (C-9, amide C=O), 135.16 (C-12), 127.99 (C-11), 122.18 (C-13), 117.55 (C-10), 103.50 (C-1'), 101.02 (C-1'''), 60.42 (C-20)

### Example 11

### 4'-Demicarosyl-8a-aza-8a-homo-10,11,12,13-tetrahydrorelomycin (Ik) and 4'-demicarosyl-9a-aza-9a-homo-10,11,12,13-tetrahydrorelomycin (Ik')

### Method C

Starting from 0.10 g (0.127 mmole) of 4'-demicarosyl-8a-aza-8a-homorelomycin (Ij) and working up as described in Example 6, Metod C, there are obtained 0.062 g (62.0%) of the product (Ik) of the following characteristics:
IR (CHCl₃) 1700, 1640 cm⁻¹
- ¹³C NMR (CDCl₃) δ ppm: 173.31 (C-1), 171.50 (C-9, amide C=O), 104.40 (C-1'), 101.02 (C-1'''), 60.42 (C-20)
Starting from 0.10 g (0.127 mmole) of 4'-demicarosyl-9a-aza-9a-homorelomycin, Ij', there are obtained according to the same method 0.63 g (63.0%) of the product (Ik') of the following characteristics:
IR (CHCl₃) 3405, 1705, 1655 cm⁻¹
- ¹³C NMR (CDCl₃) δ ppm: 177.15 (C-9, amide C=O), 173.48 (C-1), 106.75 (C-1'), 101.19 (C-1'''), 60.67 (C-20)

## Claims

1. Tylosin derivatives of the formula I 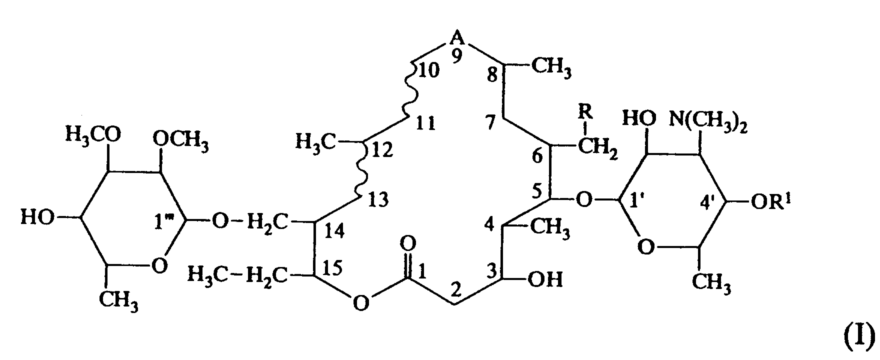 mycarosyl 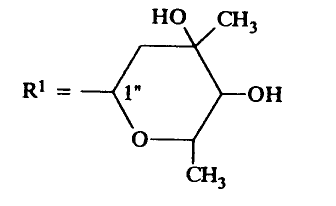 wherein A stands for a -CO-NH- or a -NH-CO- group, R stands for -CHO,-CH(OCH₃)₂ or CH₂OH, R¹ stands for mycarosyl or hydrogen and 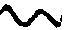 has the meaning of a double or a single bond.

2. 8a-Aza-8a-homotylosin.

3. 4'-Demicarosyl-8a-aza-8a-homotylosin.

4. 8a-Aza-8a-homo-10,11,12,13-tetrahydrotylosin.

5. 8a-Aza-8a-homotylosin dimethylacetal.

6. 4'-Demicarosyl-8a-aza-8a-homotylosin dimethylacetal.

7. 8a-Aza-8a-homo-10,11,12,13-tetrahydrotylosin dimethylacetal.

8. 8a-Aza-8a-homorelomycin.

9. 4'-Demicarosyl-8a-aza-8a-homorelomycin.

10. 8a-Aza-8a-homo-10,11,12,13-tetrahydrorelomycin.

11. 9a-Aza-9a-homorelomycin.

12. 4'-Demicarosyl-9a-aza-9a-homorelomycin.

13. 9a-Aza-9a-homo-10,11,12,13-tetrahydrorelomycin.

14. 4'-Demicarosyl-8a-aza-8a-homo-10,11,12,13-tetrahydrotylosin.

15. 4'-Demicarosyl-8a-aza-8a-homo-10,11,12,13-tetrahydrorelomycin.

16. 4'-Demicarosyl-9a-aza-9a-homo-10,11,12,13-tetrahydrorelomycin.

17. A process for preparing tylosin derivatives of the formula I 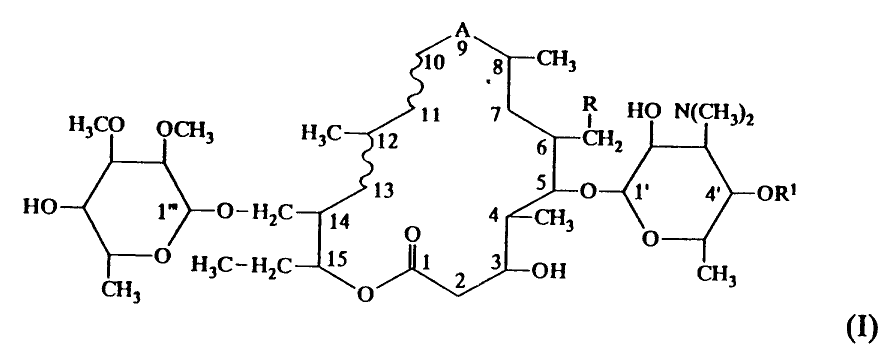 mycarosyl 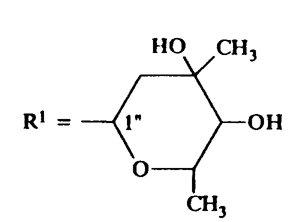 wherein
| | | | | |
|---|---|---|---|---|
| Ia | A = CO-NH, | R = CH(OCH₃)₂, | R¹ = mycarosyl, | 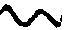 = double bond, |
| Ib | A = CO-NH, | R = CHO, | R¹ = mycarosyl, | 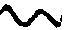 = double bond, |
| Ic | A = CO-NH, | R = CH(OCH₃)₂, | R¹ = H, | 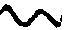 = double bond, |
| Id | A = CO-NH, | R = CHO, | R¹ = H, | 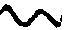 = double bond, |
| Ie | A = CO-NH, | R = CH₂OH, | R¹ = mycarosyl, | 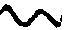 = double bond, |
| Ie' | A = NH-CO, | R = CH₂OH, | R¹ = mycarosyl, | 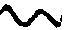 = double bond, |
| If | A = CO-NH, | R = CH₂OH, | R¹ = mycarosyl, | 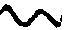 = single bond, |
| If' | A = NH-CO, | R = CH₂OH, | R¹= mycarosyl, | 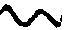 = single bond, |
| Ig | A = CO-NH, | R = CH(OCH₃)₂, | R¹ = mycarosyl, | 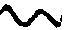 = single bond, |
| Ih | A = CO-NH, | R = CHO, | R¹ = mycarosyl, | 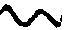 = single bond, |
| Ii | A = CO-NH, | R = CHO, | R¹ = H, | 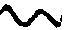 = single bond. |
| Ij | A = CO-NH, | R = CH₂OH, | R¹ = H, | 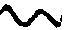 = double bond. |
| Ij' | A = NH-CO, | R = CH₂OH, | R¹ = H, | 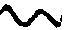 = double bond, |
| Ik | A = CO-NH, | R = CH₂OH, | R¹ =H, | 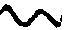 = single bond, |
| Ik' | A = NH-CO, | R = CH₂OH, | R¹ = H, | 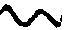 = single bond, |
*characterized in that* it comprises
A/
the Beckmann rearrangement of compounds of the formula II 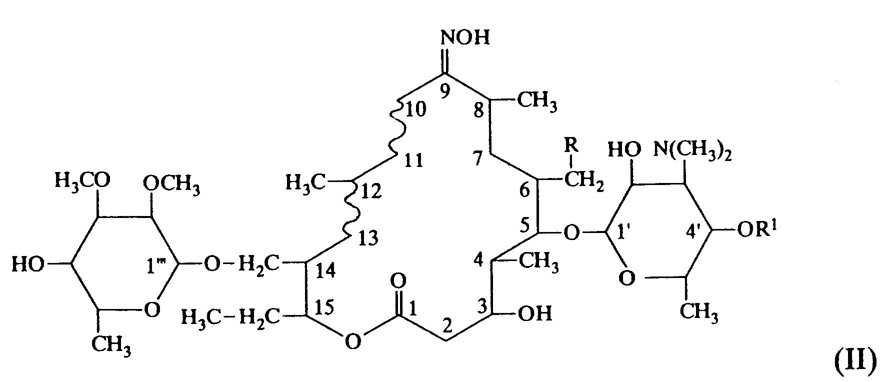 mycarosyl 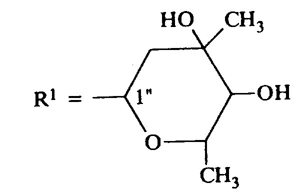 wherein
| | | | |
|---|---|---|---|
| IIa | R = CH(OCH₃)₂, | R¹ = mycarosyl, | 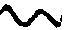 = double bond, |
| IIb | R = CHO, | R¹ = mycarosyl, | 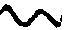 = double bond, |
| IIc | R = CH(OCH₃)₂, | R¹ = H, | 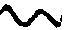 = double bond, |
| IId | R = CHO, | R¹ = H, | 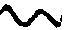 = double bond, |
| IIe | R = CH₂OH, | R¹ = mycarosyl, | 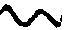 = double bond, |
| IIf | R = CH₂OH, | R¹ = mycarosyl, | 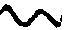 = single bond, |
| IIg | R = CH(OCH₃)₂, | R¹ = mycarosyl, | 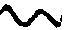 = single bond, |
| IIh | R = CHO, | R¹ = mycarosyl, | 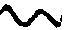 = single bond, |
with aromatic sulfochlorides of the formula 4-R-C₆H₄-SO₂Cl, wherein R represents C₁-C₃ alkyl, halo or an acylamino group of the formula -NH-COR₁, wherein R₁ represents methyl, in the presence of organic or inorganic bases such as pyridine, triethylamine or NaHCO₃, NaOH, yielding compounds of the formula I 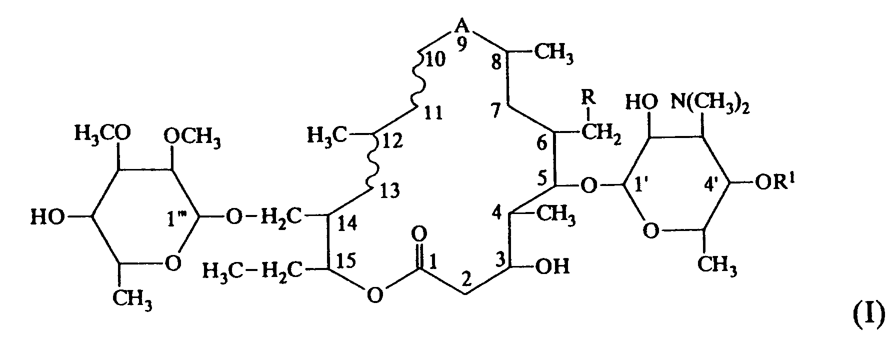 mycarosyl 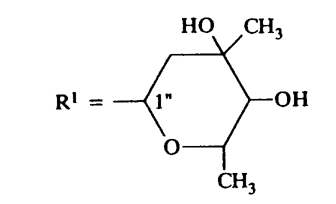 wherein
| | | | | |
|---|---|---|---|---|
| Ia | A = CO-NH, | R = CH(OCH₃)₂, | R¹ = mycarosyl, | 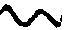 = double bond, |
| Ib | A = CO-NH, | R = CHO, | R¹ = mycarosyl, | 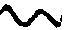 = double bond, |
| Ic | A = CO-NH, | R = CH(OCH₃)₂, | R¹ = H, | 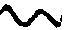 = double bond, |
| Id | A = CO-NH, | R = CHO, | R¹ = H, | 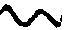 = double bond |
| Ie | A = CO-NH, | R = CH₂OH, | R¹ = mycarosyl, | 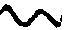 = double bond |
| Ie' | A = NH-CO, | R = CH₂OH, | R¹ = mycarosyl, | 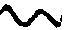 = double bond |
| If | A = CO-NH, | R = CH₂OH, | R¹ = mycarosyl, | 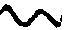 = single bond |
| If' | A = NH-CO, | R = CH₂OH, | R¹ = mycarosyl, | 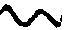 = single bond |
| Ig | A = CO-NH, | R = CH(OCH₃)₂, | R¹ = mycarosyl, | 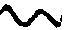 = single bond, |
| Ih | A = CO-NH, | R = CHO, | R¹ = mycarosyl, | 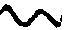 = single bond, |
B/
the hydrolysis of compounds of formula I 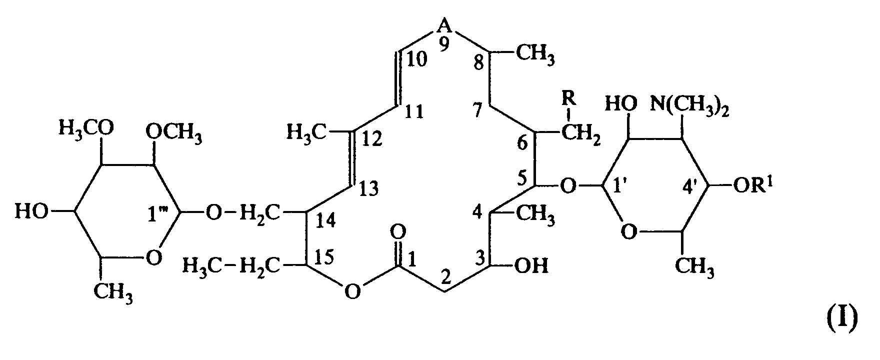 mycarosyl 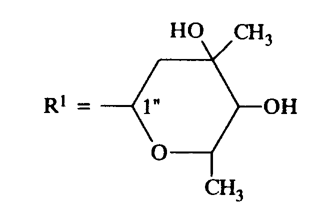 wherein
| | | | |
|---|---|---|---|
| Ia | A = CO-NH, | R = CH(OCH₃)₂, | R¹ = mycarosyl, |
| Ic | A = CO-NH, | R = CH(OCH₃)₂, | R¹ = H, |
| Ie | A = CO-NH, | R = CH₂OH, | R¹ = mycarosyl, |
| Ie' | A = NH-CO, | R = CH₂OH, | R¹ = mycarosyl, |
by means of a catalytical amount of organic acid such as trifluoroacetic acid in 50% acetonitrile or with inorganic acids such as hydrochloric or sulfuric acid in the presence of acetonitrile as solvent, yielding compounds of the formula I 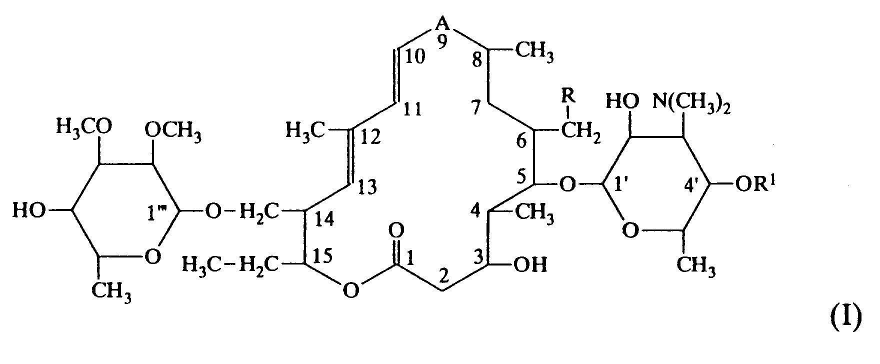 mycarosyl 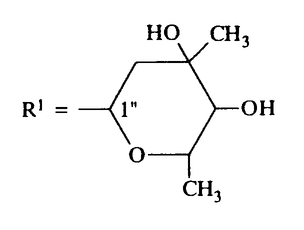 wherein
| | | | |
|---|---|---|---|
| Ib | A = CO-NH, | R = CHO, | R¹ = mycarosyl, |
| Id | A = CO-NH, | R = CHO, | R¹ = H, |
| Ij | A = CO-NH, | R = CH₂OH, | R¹ = H, |
| Ij' | A = NH-CO, | R = CH₂OH, | R¹ = H, |
C/
the catalytical hydrogenation of compounds of formula I 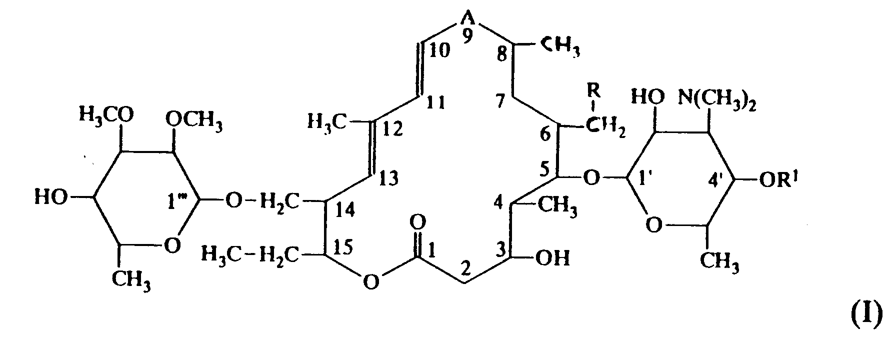 mycarosyl 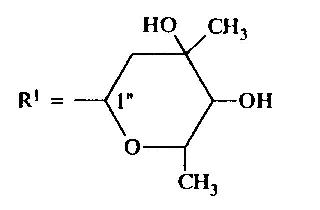
| | | | |
|---|---|---|---|
| Ia | A = CO-NH, | R = CH(OCH₃)₂, | R¹ = mycarosyl, |
| Ib | A = CO-NH, | R = CHO, | R¹ = mycarosyl, |
| Ie | A = CO-NH, | R = CH₂OH, | R¹ = mycarosyl, |
| Ie' | A = NH-CO, | R = CH₂OH, | R¹ = mycarosyl, |
| Ij | A = CO-NH, | R = CH₂OH, | R¹ = H, |
| Ij' | A = NH-CO, | R = CH₂OH, | R¹ = H, |
with H₂ in the presence of noble metal catalysts in inert solvents, such as C₁-C₄ alcohols, yielding compounds of the formula I 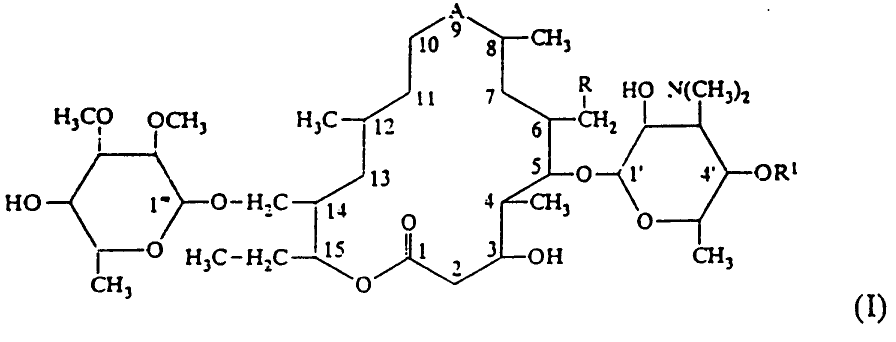 mycarosyl 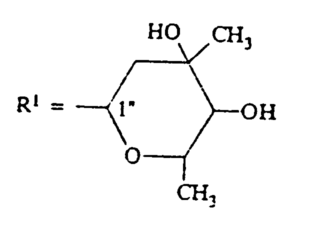 wherein
| | | | |
|---|---|---|---|
| If | A = CO-NH, | R = CH₂OH, | R¹ = mycarosyl, |
| If' | A = NH-CO, | R = CH₂OH, | R¹ = mycarosyl, |
| Ig | A = CO-NH, | R = CH(OCH₃)₂, | R¹ = mycarosyl, |
| Ih | A = CO-NH, | R = CHO, | R¹ = mycarosyl, |
| Ii | A = CO-NH, | R = CHO, | R¹ = H, |
| Ik | A = CO-NH, | R = CH₂OH, | R¹ = H, |
| Ik' | A = NH-CO, | R = CH₂OH, | R¹ = H. |

## Patentansprüche

1. Tylosinderivate der Formel I 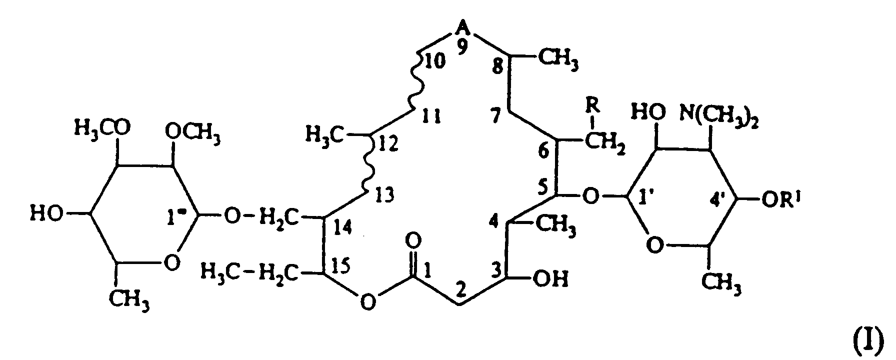 Mycarosyl 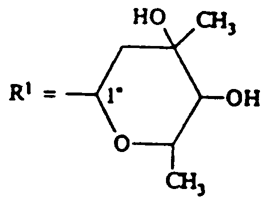 worin A für eine -CO-NH- oder eine -NH-CO-Gruppe steht, R für -CHO, -CH(OCH₃)₂ oder CH₂OH steht, R² für Mycarosyl oder Wasserstoff steht und 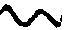 die Bedeutung einer Doppel- oder Einfachbindung hat.

2. 8a-Aza-8a-homotylosin.

3. 4'-Demicarosyl-8a-aza-8a-homotylosin.

4. 8a-Aza-8a-home-10,11,12,13-tetrahydrotylosin.

5. 8a-Aza-8a-homotylosin-dimethylacetal.

6. 4'-Demicarosyl-8a-aza-8a-homotylosin-dimethylacetal.

7. 8a-Aza-8a-homo-10,11,12,12-tetrahydrotylosin dimethylacetal.

8. 8a-Aza-8a-homorelomycin.

9. 4'-Demicarosyl-8a-aza-8a-homorelomycin.

10. 8a-Aza-8a-homo-10,11,12,13-tetrahydrorelomycin.

11. 9a-Aza-9a-homorelomycin.

12. 4'-Demicarosyl-9a-aza-9a-homorelomycin.

13. 9a-Aza-9a-homo-10,11,12,13-tetrahydrorelomycin.

14. 4'-Demicarosyl-8a-aza-8a-homo-10,11,12,13-tetrahydrotylosin.

15. 4'-Demicarosyl-8a-aza-8a-homo-10,11,12,13-tetrahydrorelomycin.

16. 4'-Demicarosyl-9a-aza-9a-homo-10,11,12,13-tetrahydrorelomycin.

17. Verfahren zur Herstellung von Tylosinderivaten der Formel I 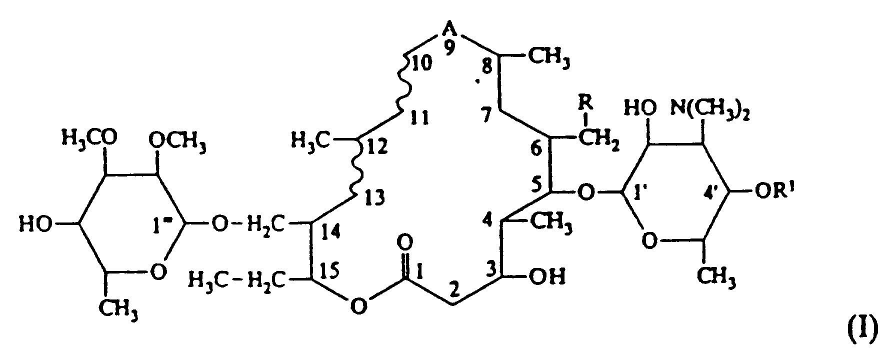 Mycarosyl 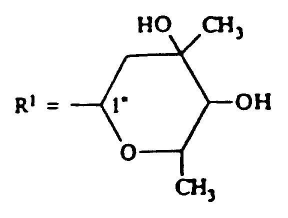 worin
| | | | | |
|---|---|---|---|---|
| Ia | A = CO-NH, | R = CH(OCH₃)₂, | R¹ = Mycarosyl, | 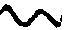 = Doppelbindung, |
| Ib | A = CO-NH, | R = CHO, | R¹ = Mycarosyl, | 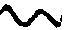 = Doppelbindung, |
| Ic | A = CO-NH, | R = CH(OCH₃)₂, | R¹ = H, | 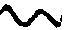 = Doppelbindung, |
| Id | A = CO-NH, | R = CHO, | R¹ = H, | 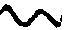 = Doppelbindung, |
| Ie | A = CO-NH, | R = CH₂OH, | R¹ = Mycarosyl, | 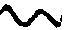 = Doppelbindung, |
| Ie' | A = NH-CO, | R = CH₂OH, | R¹ = Mycarosyl, | 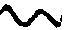 = Doppelbindung, |
| If | A = CO-NH, | R = CH₂OH, | R¹ = Mycarosyl, | 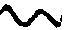 = Einfachbindung, |
| If' | A = NH-CO, | R = CH₂OH, | R¹ = Mycarosyl, | 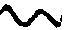 = Einfachbindung, |
| Ig | A = CO-NH, | R = CH(OCH₃)₂, | R¹ = Mycarosyl, | 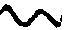 = Einfachbindung, |
| Ih | A = CO-NH, | R = CHO, | R¹ = Mycarosyl, | 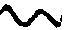 = Einfachbindung, |
| Ii | A = CO-NH, | R = CHO, | R¹ = H, | 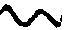 = Einfachbindung, |
| Ij | A = CO-NH, | R = CH₂OH, | R¹ = H, | 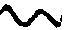 = Doppelbindung, |
| Ij' | A = NH-CO, | R = CH₂OH, | R¹ = H, | 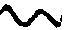 = Doppelbindung, |
| Ik | A = CO-NH, | R = CH₂OH, | R¹ = H, | 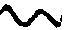 = Einfachbindung, |
| Ik' | A = NH-CO, | R = CH₂OH, | R¹ = H, | 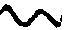 = Einfachbindung, |
dadurch gekennzeichnet, dass
A/
durch die Beckmann-Umlagerung von Verbindungen der Formel II 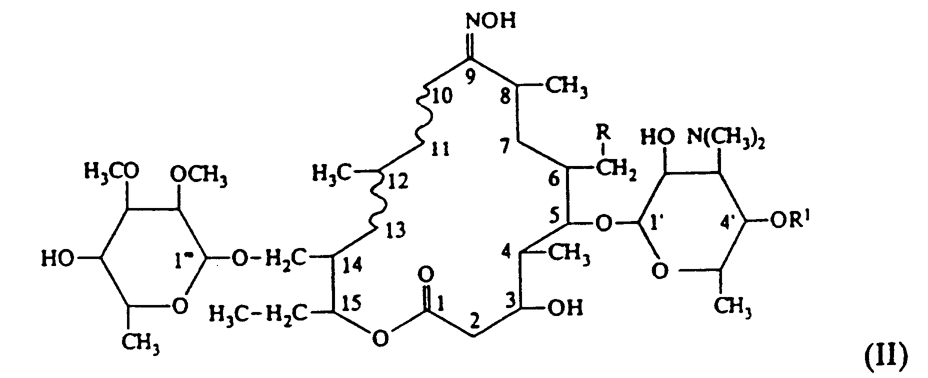 Mycarosyl 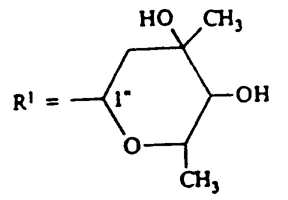 worin
| | | | |
|---|---|---|---|
| IIa | R = CH(OCH₃)₂, | R¹ = Mycarosyl, | 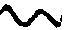 = Doppelbindung, |
| IIb | R = CHO, | R¹ = Mycarosyl, | 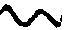 = Doppelbindung, |
| IIc | R = CH(OCH₃)₂, | R¹ = H, | 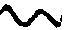 = Doppelbindung, |
| IId | R = CHO, | R¹ = H, | 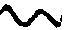 = Doppelbindung, |
| IIe | R = CH₂OH, | R¹ = Mycarosyl, | 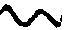 = Doppelbindung, |
| IIf | R = CH₂OH, | R¹ = Mycarosyl, | 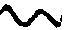 = Einfachbindung, |
| IIg | R = CH(OCH₃)₂, | R¹ = Mycarosyl, | 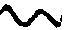 = Einfachbindung, |
| IIh | R = CHO, | R¹ = Mycarosyl, | 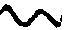 = Einfachbindung, |
mit aromatischen Sulfochloriden der Formel 4-R-C₆H₄-SO₂Cl, worin R die Bedeutung von C₁-C₃-Alkyl, Halogen oder einer Arylaminogruppe der Formel -NH-COR₁ hat, worin R₁ Methyl bedeutet, in Anwesenheit von organischen oder anorganischen Basen wie Pyridin, Triäthylamin oder NaHCO₃, NaOH, Verbindungen der Formel I 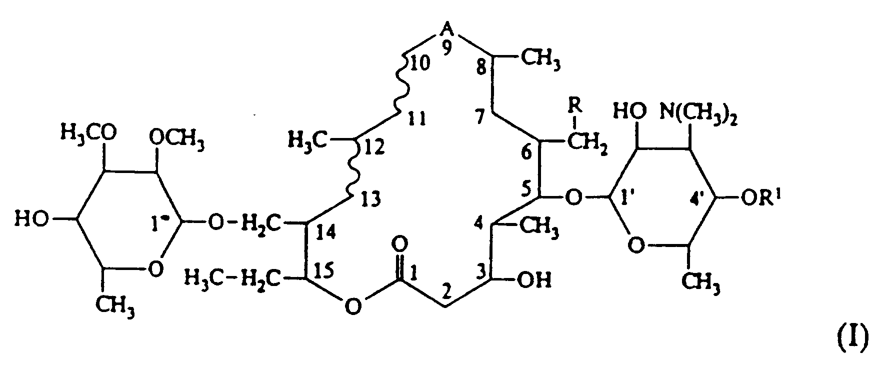 Mycarosyl 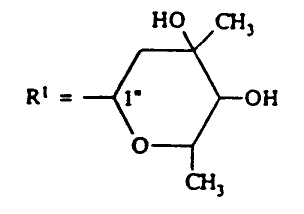 worin
| | | | | |
|---|---|---|---|---|
| Ia | A = CO-NH, | R = CH(OCH₃)₂, | R¹ = Mycarosyl, | 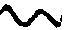 = Doppelbindung, |
| Ib | A = CO-NH, | R = CHO, | R¹ = Mycarosyl, | 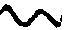 = Doppelbindung, |
| Ic | A = CO-NH, | R = CH(OCH₃)₂, | R¹ = H, | 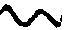 = Doppelbindung, |
| Id | A = CO-NH, | R = CHO, | R¹ = H, | 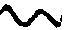 = Doppelbindung, |
| Ie | A = CO-NH, | R = CH₂OH, | R¹ = Mycarosyl, | 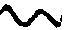 = Doppelbindung, |
| Ie' | A = NH-CO, | R = CH₂OH, | R¹ = Mycarosyl, | 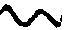 = Doppelbindung, |
| If | A = CO-NH, | R = CH₂OH, | R¹ = Mycarosyl, | 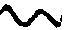 = Einfachbindung, |
| If' | A = NH-CO, | R = CH₂OH, | R¹ = Mycarosyl, | 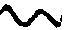 = Einfachbindung, |
| Ig | A = CO-NH, | R = CH(OCH₃)₂, | R¹ = Mycarosyl, | 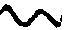 = Einfachbindung, |
| Ih | A = CO-NH, | R = CHO, | R¹ = Mycarosyl, | 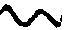 = Einfachbindung, |
erhalten werden,
B/
durch Hydrolyse der Verbindungen der Formel I 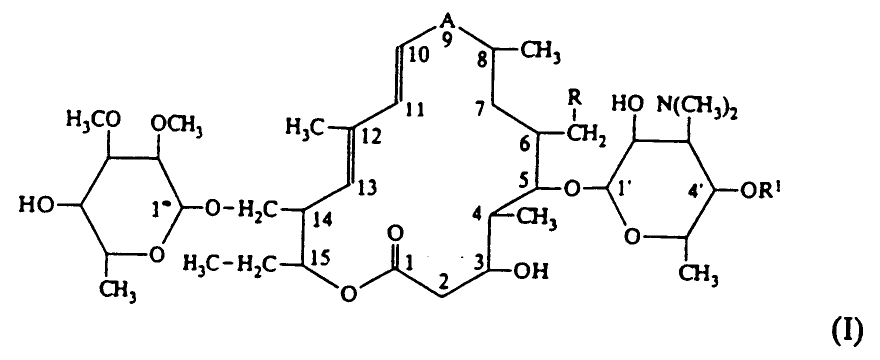 Mycarosyl 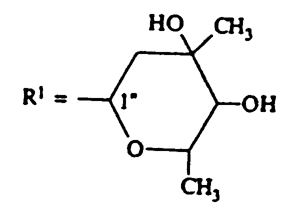 worin
| | | | |
|---|---|---|---|
| Ia | A = CO-NH, | R = CH(OCH₃)₂, | R¹ = Mycarosyl, |
| Ic | A = CO-NH, | R = CH(OCH₃)₂, | R¹ = H, |
| Ie | A = CO-NH, | R = CH₂OH, | R¹ = Mycarosyl, |
| Ie' | A = NH-CO, | R = CH₂OH, | R¹ = Mycarosyl, |
mittels einer katalytischen Menge von organischen Säuren wie Trifluoressigsäure in 50% Acetonitril oder mit anorganischen Saüren wie Salsaüre oder Schwefelsäure in Anwesenheit von Acetonitril als Lösungsmittel Verbindungen der Formel I 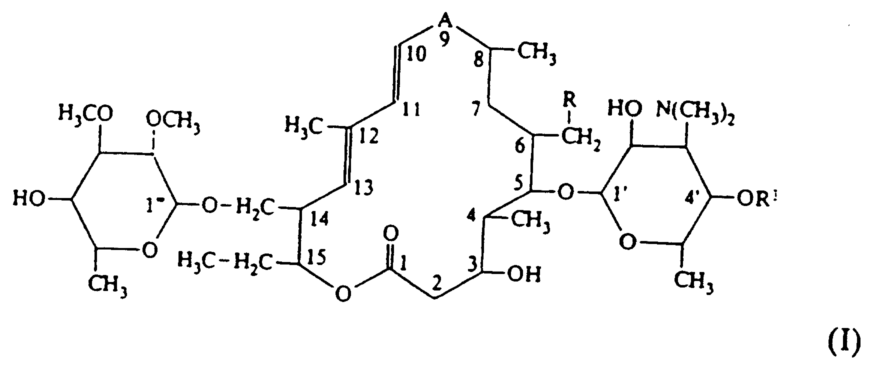 Mycarosyl 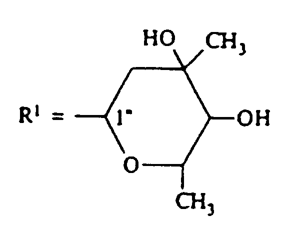 worin
| | | | |
|---|---|---|---|
| Ib | A = CO-NH, | R = CHO, | R¹ = Mycarosyl, |
| Id | A = CO-NH, | R = CHO, | R¹ = H, |
| Ij | A = CO-NH, | R = CH₂OH, | R¹ = H, |
| Ij' | A = NH-CO, | R = CH₂OH, | R¹ = H, |
erhalten werden,
C/
durch katalytische Hydrierung von Verbindungen der Formel I 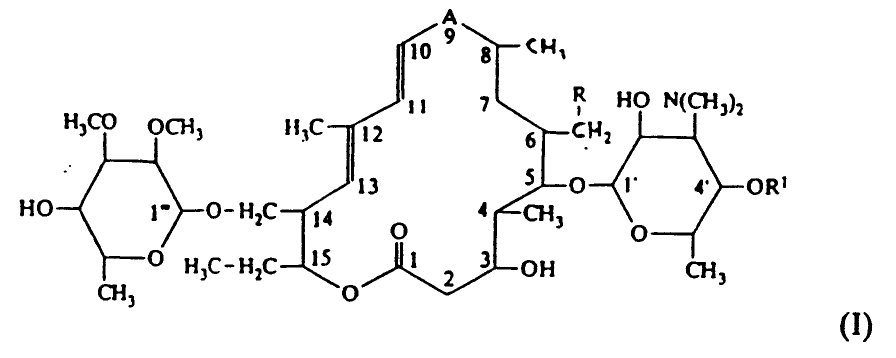 Mycarosyl 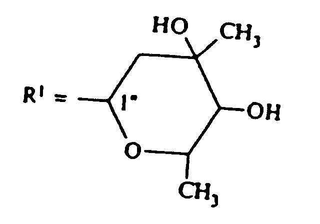 worin
| | | | |
|---|---|---|---|
| Ia | A = CO-NH, | R = CH(OCH₃)₂, | R¹ = Mycarosyl, |
| Ib | A = CO-NH, | R = CHO, | R¹ = Mycarosyl, |
| Ie | A = CO-NH, | R = CH₂OH, | R¹ = Mycarosyl, |
| Ie' | A = NH-CO, | R = CH₂OH, | R¹ = Mycarosyl, |
| Ij | A = CO-NH, | R = CH₂OH, | R¹ = H, |
| Ij' | A = NH-CO, | R = CH₂OH, | R¹ = H, |
mit H₂ in Anwesenheit von Edelmetallkatalysatoren in inerten Lösungsmitteln wie C₁-C₄ Alkoholen die Verbindungen der Formel I 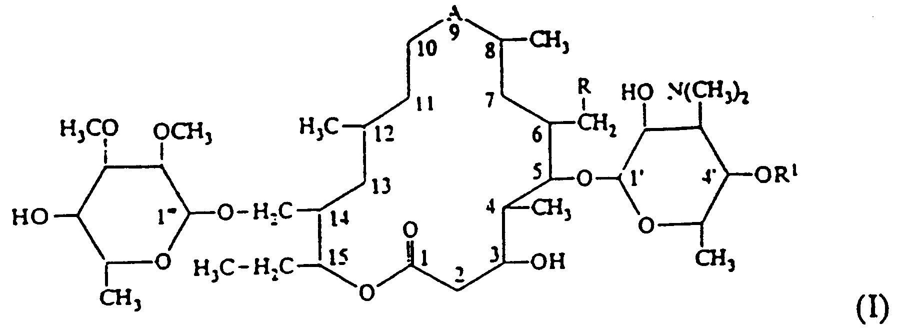 Mycarosyl 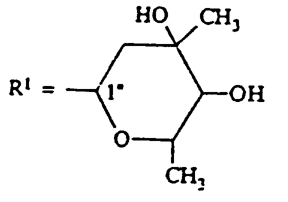 worin
| | | | |
|---|---|---|---|
| If | A = CO-NH, | R = CH₂OH, | R¹ = Mycarosyl, |
| If' | A = NH-CO, | R = CH₂OH, | R¹ = Mycarosyl, |
| Ig | A = CO-NH, | R = CH(OCH₃)₂, | R¹ = Mycarosyl, |
| Ih | A = CO-NH, | R = CHO, | R¹ = Mycarosyl, |
| Ii | A = CO-NH, | R = CHO, | R¹ = H, |
| Ik | A = CO-NH, | R = CH₂OH, | R¹ = H, |
| Ik' | A = NH-CO, | R = CH₂OH, | R¹ = H, |
erhalten werden.

## Revendications

1. Dérivés de la tylosine répondant à la formule I 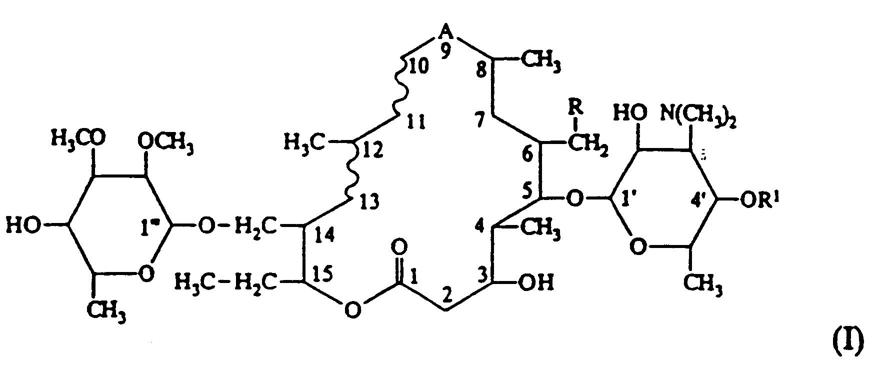 mycarosyle 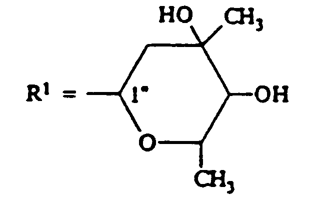 dans laquelle A représente un groupe -CO-NH- ou un groupe -NH-CO, R représente un groupe -CHO, -CH(OCH₃)₂ ou -CH₂OH, R¹ représente un groupe mycarosyle ou un atome d'hydrogène et 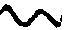 désigne une double liaison ou un simple liaison.

2. 8a-Aza-8a-homotylosine.

3. 4'-Démycarosyl-8a-aza-8a-homotylosine.

4. 8a-Aza-8a-homo-10,11,12,13-tétrahydrotylosine.

5. 8a-Aza-8a-homotylosine diméthylacétal.

6. 4'-Démycarosyl-8a-aza-8a-homotylosinediméthylacétal.

7. 8a-Aza-8a-homo-10,11,12,13-tétrahydrotylosine diméthylacétal.

8. 8a-Aza-8a-homorélomycine.

9. 4'-Démycarosyl-8a-aza-8a-homorélomycine.

10. 8a-Aza-8a-homo-10,11,12,13-tétrahydrorélomycine.

11. 9a-Aza-9a-homorélomycine.

12. 4'-Démycarosyl-9a-aza-9a-homorélomycine.

13. 9a-Aza-9a-homo-10,11,12,13-tétrahydrorélomycine.

14. 4'-Démycarosyl-8a-aza-8a-homo-10,11,12,13-tétrahydrotylosine.

15. 4'-Démycarosyl-8a-aza-8a-homo-10,11,12,13-tétrahydrorélomycine.

16. 4'-Démycarosyl-9a-aza-9a-homo-10,11,12,13-tétrahydrorélomycine.

17. Procédé de préparation de dérivés de la tylosine de la formule 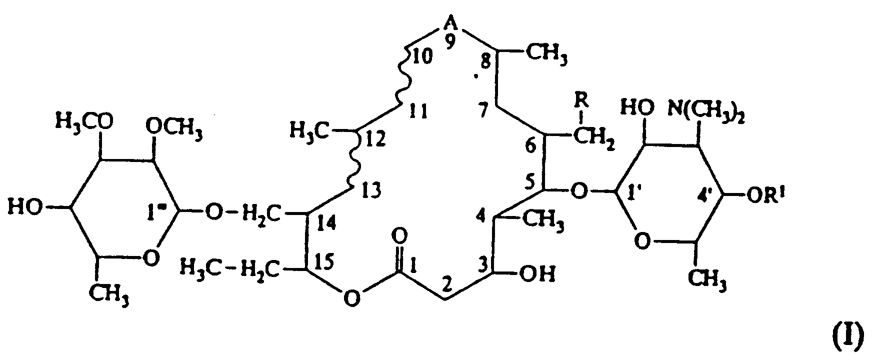 mycarosyle 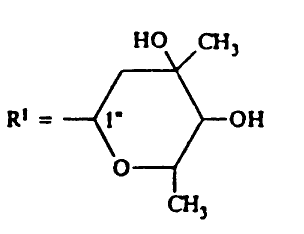 dans laquelle
| | | | | |
|---|---|---|---|---|
| Ia | A = CO-NH, | R = CH(OCH₃)₂, | R¹ = mycarosyle, | 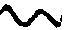 = double liaison, |
| Ib | A = CO-NH, | R= CHO, | R¹ = mycarosyle, | 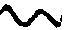 = double liaison, |
| Ic | A = CO-NH, | R = CH(OCH₃)₂, | R¹ = H, | 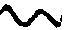 = double liaison, |
| Id | A = CO-NH, | R = CHO, | R¹ = H, | 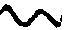 = double liaison, |
| Ie | A = CO-NH, | R = CH₂OH, | R¹ = mycarosyle, | 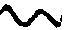 = double liaison, |
| Ie' | A = NH-CO, | R = CH₂OH, | R¹ = mycarosyle, | 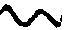 = double liaison, |
| If | A - CO-NH, | R = CH₂OH, | R¹ = mycarosyle | 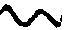 = simple liaison, |
| If' | A = NH-CO, | R = CH₂OH, | R¹ = mycarosyle, | 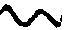 = simple liaison, |
| Ig | A = CO-NH, | R = CH(OCH₃)₂, | R¹ = mycarosyle, | 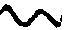 = simple liaison, |
| Ih | A = CO-NH, | R = CHO, | R¹ = mycarosyle, | 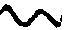 = simple liaison, |
| Ii | A = CO-NH, | R = CHO, | R¹ = H, | 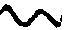 = simple liaison, |
| Ij | A = CO-NH, | R = CH₂OH, | R¹ = H, | 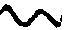 = double liaison, |
| Ij' | A = NH-CO, | R = CH₂OH, | R¹ = H, | 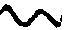 = double liaison, |
| Ik | A = CO-NH, | R = CH₂OH, | R¹ = H, | 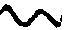 = simple liaison, |
| Ik' | A = NH-CO, | R = CH₂OH, | R¹ = H, | 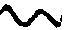 = simple liaison, |
caractérisé en ce qu'il comprend
A/
la transposition de Beckmann de composés de la formule II 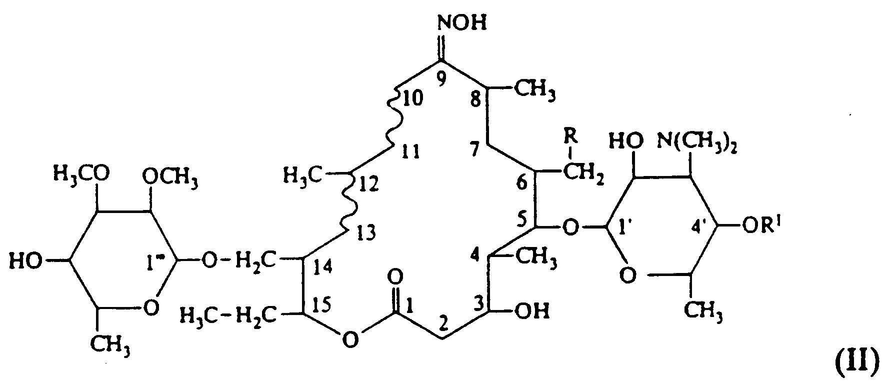 mycarosyle 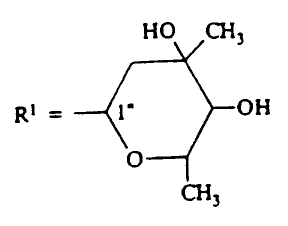 dans laquelle
| | | | |
|---|---|---|---|
| IIa | R = CH(OCH₃)₂, | R¹ = mycarosyle, | 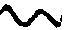 = double liaison, |
| IIb | R = CHO, | R¹ = mycarosyle, | 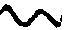 = double liaison, |
| IIc | R = CH(OCH₃)₂, | R¹ = H, | 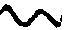 = double liaison, |
| IId | R = CHO, | R¹ = H, | 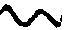 = double liaison, |
| IIe | R = CH₂OH, | R¹ = mycarosyle, | 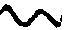 = double liaison, |
| IIf | R = CH₂OH, | R¹ = mycarosyle, | 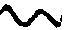 = simple liaison, |
| IIg | R = CH(OCH₃)₂, | R¹ = mycarosyle, | 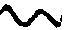 = simple liaison, |
| IIh | R = CHO, | R¹ = mycarosyle, | 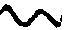 = simple liaison, |
avec des sulfochlorures aromatiques de la formule 4-R-C₆H₄-SO₂Cl dans laquelle R représente un groupe alkyle en C₁ à C₃, un atome d'halogène ou un radical acylamino de la formule -NH-COR₁, dans laquelle R₁ représente le radical méthyle, en présence de bases organiques ou inorganiques, comme la pyridine, la triéthylamine, ou NaHCO₃, NaOH, de façon à obtenir des composés de la formule I 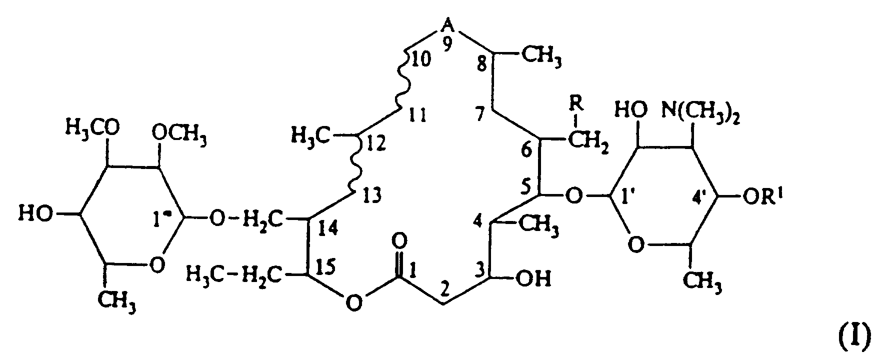 mycarosyle 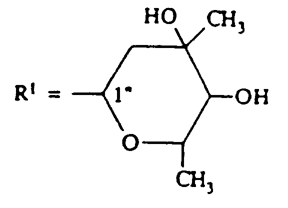 dans laquelle
| | | | | |
|---|---|---|---|---|
| Ia | A = CO-NH, | R = CH(OCH₃)₂, | R¹ = mycarosyle, | 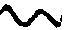 = double liaison, |
| Ib | A = CO-NH, | R = CHO, | R¹ = mycarosyle, | 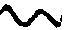 = double liaison, |
| Ic | A = CO-NH, | R = CH(OCH₃)₂, | R¹ = H, | 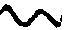 = double liaison, |
| Id | A = CO-NH, | R = CHO, | R¹ = H, | 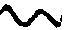 = double liaison, |
| Ie | A = CO-NH, | R = CH₂OH, | R¹ = mycarosyle, | 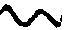 = double liaison, |
| Ie' | A = NH-CO, | R = CH₂OH, | R¹ = mycarosyle, | 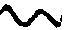 = double liaison, |
| If | A = CO-NH, | R = CH₂OH, | R¹ = mycarosyle, | 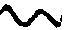 = simple liaison, |
| If' | A = NH-CO, | R = CH₂OH, | R¹ = mycarosyle, | 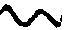 = simple liaison, |
| Ig | A = CH-NH, | R = CH(OCH₃)₂, | R¹ = mycarosyle, | 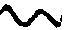 = simple liaison, |
| Ih | A = CO-NH, | R = CHO, | R¹ = mycarosyle, | 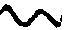 = simple liaison, |
B/
l'hydrolyse de composés de la formule I 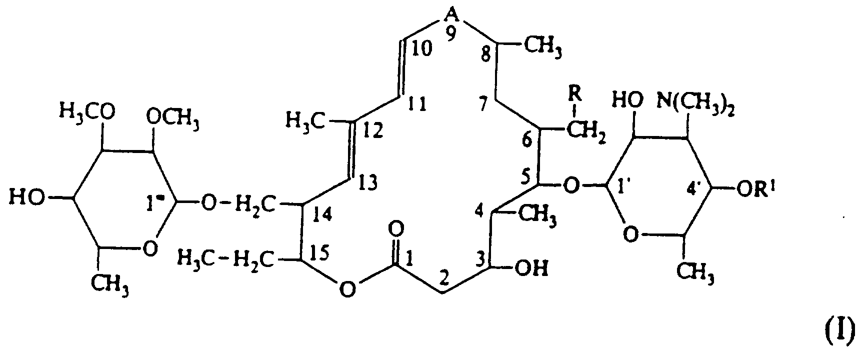 mycarosyle 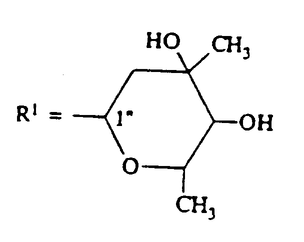 dans laquelle
| | | | |
|---|---|---|---|
| Ia | A = CO-NH, | R = CH(OCH₃)₂, | R¹ = mycarosyle, |
| Ic | A = CO-NH, | R = CH(OCH₃)₂, | R¹ = H, |
| Ie | A = CO-NH, | R = CH₂OH, | R¹ = mycarosyle, |
| Ie' | A = NH-CO, | R = CH₂OH, | R¹ = mycarosyle, |
à l'aide d'une proportion catalytique d'un acide organique, comme l'acide trifluoracétique dans 50% d'acétonitrile ou à l'aide d'acides inorganiques, tels que l'acide chlorhydrique ou l'acide sulfurique, en présence d'acétonitrile servant de solvant, de façon à obtenir des composés de la formule I 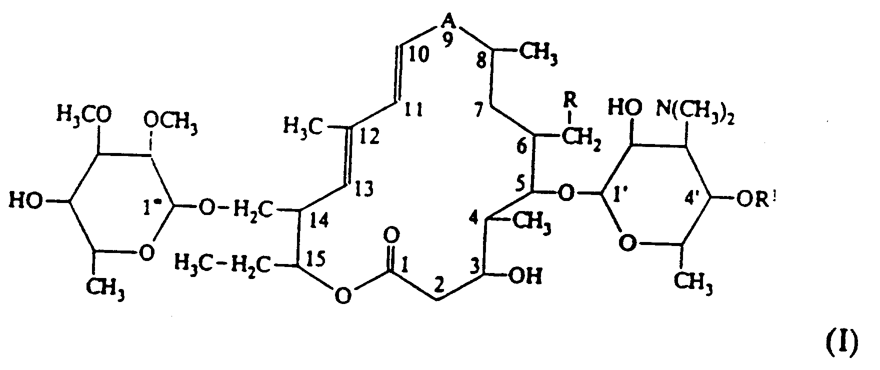 mycarosyle 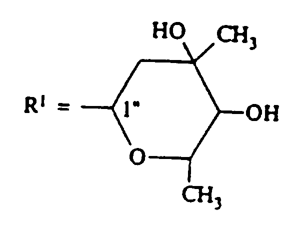 dans laquelle
| | | | |
|---|---|---|---|
| Ib | A = CO-NH, | R = CHO, | R¹ = mycarosyle, |
| Id | A = CO-NH, | R = CHO, | R¹ = H, |
| Ij | A = CO-NH, | R = CH₂OH, | R¹ = H, |
| Ij' | A = NH-CO, | R = CH₂OH, | R¹ = H, |
C/
l'hydrogénation catalytique de composés de la formule I 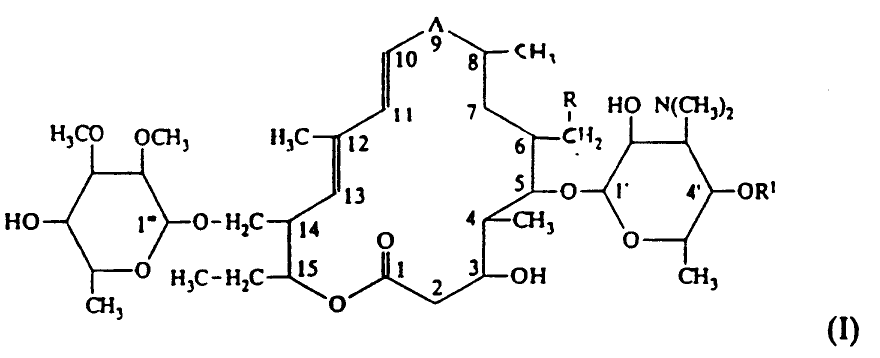 mycarosyle 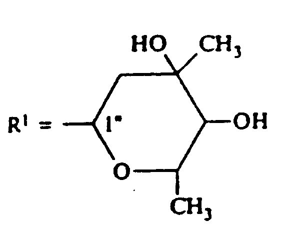
| | | | |
|---|---|---|---|
| Ia | A = CO-NH, | R = CHO(OCH₃)₂, | R¹ = mycarosyle, |
| Ib | A = CO-NH, | R = CHO, | R¹ = mycarosyle, |
| Ie | A = CO-NH, | R = CH₂OH, | R¹ = mycarosyle, |
| Ie' | A = NH-CO, | R = CH₂OH, | R¹ = mycarosyle, |
| Ij | A = CO-NH, | R = CH₂OH, | R¹ = H, |
| Ij' | A = NH-CO, | R = CH₂OH, | R¹ = H, |
avec H₂ en présence de catalyseurs à base de métaux nobles, dans des solvants inertes tels que des alcools en C₁ à C₄, de façon à obtenir des composés de la formule I 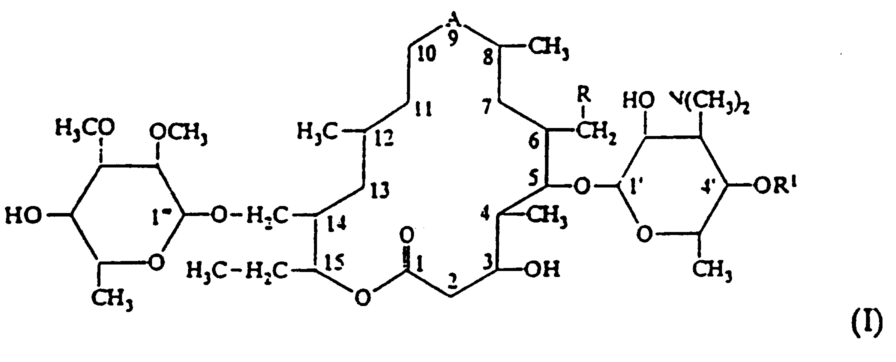 mycarosyle 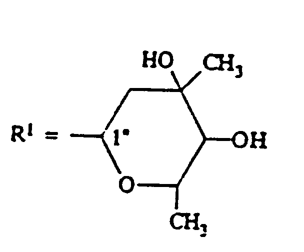 dans laquelle
| | | | |
|---|---|---|---|
| If | A = CO-NH, | R = CHO₂OH, | R¹ = mycarosyle, |
| If' | A = NH-CO, | R = CH₂OH, | R¹ = mycarosyle, |
| Ig | A = CO-NH, | R = CH(OCH₃)₂, | R¹ = mycarosyle, |
| Ih | A = CO-NH, | R = CHO, | R¹ = mycarosyle, |
| Ii | A = CO-NH, | R = CHO, | R¹ = H, |
| Ik | A = CO-NH, | R = CH₂OH, | R¹ = H, |
| Ik' | A = NH-CO, | R = CH₂OH, | R¹ = H, |
